# FASCICULE DE BREVET EUROPEEN

(11) **EP 0 682 944 B1**
(45) Date de publication et mention de la délivrance du brevet: **07.04.1999**
(21) Numéro de dépôt: 95401012.0
(22) Date de dépôt: 02.05.1995
(51) Int. Cl.: A61K 9/14, A61K 47/26, A61K 47/18, A61K 47/00

(54) **Formulation stable lyophilisée comprenant une protéine; kit de dosage**
Protein enthaltende stabilisierte Formulierung und Dosierungskit
Stable lyophilized formulation containing a protein and dosage kit

(30) Priorité: 04.05.1994 FR 9405486
(43) Date de publication de la demande: 22.11.1995
(73) Titulaire: SANOFI, 75008 Paris (FR)
(72) Inventeur: Bayol, Alain, F-31170 Tournefeuille (FR); Breul, Thierry, F-34080 Montpellier (FR); Dupin, Patrice, F-31520 Ramonville Saint Agne (FR); Faure, Philippe, F-34970 Maurin (FR)
(74) Mandataire: Le Guen, Gérard

(56) Documents cités:
- WO-A-93/19776
- DATABASE WPI Section Ch, Week 9020 Derwent Publications Ltd., London, GB; Class B04, AN 90-151822 & JP-A-02 096 536 (GREEN CROSS CORPORATION) , 9 Avril 1990
- CHEMICAL & PHARMACEUTICAL BULLETIN, vol. 42, no. 1, Janvier 1994 TOKYO, pages 5-8, XP 000425037 IZUTSU K. ET AL 'EFFECT OF MANNITOL CRYSTALLINITY ON THE STABILISATION OF ENZYMES DURING FREEZE-DRYING 000425037'

## Description

La présente invention concerne une formulation pharmaceutiquement acceptable se présentant sous forme de lyophilisat et contenant une protéine en tant que principe actif. Cette formulation est stable à 25°C et peut être reconstituée sous forme liquide par ajout d'un solvant. Elle peut être administrée par voie parentérale à l'homme ou à l'animal par voie sous-cutanée, intraveineuse, ou intramusculaire, ou utilisée dans une trousse de dosage sous la forme d'un kit de dosage

On sait que la formulation a un effet considérable sur la dégradation des protéines en cours de lyophilisation, ainsi qu'un fort impact sur leur stabilité sous forme lyophilisée. Les diverses variables de formulation qui affectent ces paramètres sont principalement le pH, la quantité de sels présents, le type et la quantité d'excipients, le type de cryoprotection choisi, ainsi que les températures, pression et temps des opérations de congélation, sublimation et dessiccation choisis. Ces différentes variables influent sur l'état physique du lyophilisat obtenu, à savoir : amorphe vitreux, amorphe mou, cristallin ou une combinaison de ces états.

Le rôle de chacune de ces variables a été étudié séparément, mais leur effet synergique est encore mal élucidé (Pikal MJ., Dellermann KM., Roy ML. Riggin MN., The effects of formulation variables on the stability of freeze-dried Human Growth Hormone, Pharm. Research., 1991, *8*, N° 4, 427-436).

Une revue bibliographique sur l'influence des acides aminés et des polyols sur les propriétés des solutions à lyophiliser ou des lyophilisats a permis de dégager les conclusions suivantes :
Les avantages et les inconvénients liés à la présence d'acides aminés, de mannitol d'une phase cristalline ou d'une phase amorphe sont répertoriés ci-après :

### Avantages liés à la présence d'acides aminés.

Il a été démontré que la présence de glycine dans un lyophilisat, induisait une cristallisation des molécules présentes en solution au cours de l'étape de congélation de la lyophilisation (Korey DJ., Schwartz JB, Effects of excipients on the cristallization of pharmaceutical compounds during lyophylization, J. Parenteral Sci. Tech., 1989, *43*, 2, 80-83). Cette cristallisation du principe actif néanmoins peu probable dans le cas des protéines permet d'améliorer sa stabilité.

L'alanine sous forme cristallisée a l'avantage d'empêcher l'affaissement du lyophilisat en cours de sublimation et de dessiccation et de permettre l'obtention d'un lyophilisat d'une surface spécifique plus importante et permet donc une dessiccation plus rapide (Pikal MJ., Freeze-drying of proteins, Biopharm. 26-30 october 1990).

### Inconvénients liés à la présence d'acides aminés.

L'ajout d'un acide aminé à un sucre ou à un polyol dans une solution à lyophiliser a généralement pour effet de diminuer la température de transition vitreuse du sucre (te Booy MPWM de Ruiter RA., de Meere ALJ., Evaluation of the physical stability of freeze-dried sucrose containing formulations by differential scanning calorimetry, Pharm. Research., 1992, *9*, 109-114). Or un abaissement de température de transition vitreuse est généralement synonyme de moindre stabilité d'un lyophilisat (Franks F., Freeze-drying ; from empiricism to predictability, Cryo-letters, 1990, *11*, 93-110).

### Avantages liés à la présence de mannitol.

La présence de mannitol sous forme amorphe englobant la protéine garantit la présence d'eau non cristallisée liée à la protéine, en cours de congélation, et empêche de ce fait la dénaturation de la protéine. De plus la présence de polyols stabilise les protéines contre les dégradations thermiques, par interactions hydrophobes (Back JF, Oakenfull D., Smith MB., Increased thermal stability of proteins in the presence of sugars and polyols, Biochemistry, 1979, *18*, 23, 5191-96).

### Inconvénients liés à la présence de mannitol.

Il a été rapporté que le mannitol ne permettait pas de conserver l'activité d'une enzyme à 37°C, contrairement au lactose. (Ford AW, Dawson PJ., The effect of carbohydrate additives in the freeze-drying of alkaline phosphatase, J. Pharm. Pharmacol., 1993, *45* (2), 86-93).

### Avantages liés à la présence d'une phase cristalline.

La présence d'un soluté cristallisé, dans une solution congelée est un moyen de stabiliser les protéines en cours de dessiccation (Carpenter JF. & Crowe JH., Modes of stabilization of a protein by organic solutes during dessiccation, Cryobiology, 1988, *25*, 459-470).

### Inconvénients liés à la présence d'une phase cristalline.

Il a été démontré que la perte d'activité d'une protéine lyophilisée était directement reliée aux taux de cristallinité de la molécule cryoprotectrice (Izutsu KL, Yoshioka S., Terao T., Decreased protein-stabilizing effects of cryoprotectants due to crystallization., Pharm. Research. 1993, *10*, N° 8, 1232-1237).

Dans la formulation des médicaments contenant des protéines, la cristallisation des excipients doit être évitée selon : (Hermansky M., Pesak M., Lyophilization of drugs. VI Amorphous and Cristalline forms Cesk. Farm., 1993, *42*, (2), 95-98).

### Avantages liés à la présence d'une phase amorphe.

La présence d'additifs à l'état amorphe stabilise l'activité de certaines enzymes proportionnellement à la concentration de l'additif selon (Izutsu KL, Yoshioka S., Terao T., Decreased protein-stabilizing effects of cryoprotectants due to crystallization., Pharm. Research. 1993, *10*, N° 8, 1232-1237).

Il a également été montré que le maintien de la phase amorphe durant la congélation des protéines en présence de mannitol était perturbé lorsque celui-ci se trouve sous une forme cristallisée (Izutsu K.L, Yoshioka S., Terao T., Effect of Mannitol Crystallinity on the Stabilization of Enzymes during Freeze-Drying, Chem.Pharm. Bull. (1994) 42(1) 5-8).

L'effet cryoprotecteur des excipients est attribué à l'état amorphe de la glycine dans le lyophilisat obtenu (Pikal MJ., Dellermann KM., Roy ML. Riggin MN., The effects of formulation variables on the stability of freeze-dried Human Growth Hormone, Pharm. Research., 1991, *8*, N° 4, 427-436).

### Inconvénients liés à la présence d'une phase amorphe.

En présence d'une phase amorphe solide seule, le lyophilisat s'affaisse aux températures supérieures à la température de transition vitreuse en cours de congélation.

Au sein d'une phase amorphe molle les réactions chimiques de dégradation ont une cinétique beaucoup plus rapide qu'au sein d'une phase cristalline.

En conclusion, la revue exhaustive de la littérature scientifique concernant l'effet des excipients sur la stabilisation des protéines permet de trouver des informations contradictoires sur leurs propriétés. Aucune théorie sur les relations entre la structure d'un lyophilisat et sa stabilité ne fait l'unanimité. De même, le rôle des polyols et des acides aminés, seuls ou en association, n'est pas décrit selon un ensemble de propriétés généralisables, mais a été observé avec des résultats contradictoires selon les protéines étudiées et les quantités d'excipients mises en jeu.

Par exemple, on a pu montrer que l'albumine, dans une gamme de concentration déterminée, et en présence d'un sucre alcoolique et d'un acide aminé basique, modifiait la solubilité de préparations de plasminogène et leur stabilité à l'état dissous lorsque celles-ci étaient soumises à des conditions sévères (traitement thermique d'inactivation virale...) (JP-A-2 096 536).

Il a donc maintenant été trouvé, de façon tout-à-fait surprenante, un effet synergique entre le mannitol et l'alanine sur la stabilisation des protéines lyophilisées. Il a été démontré que cet effet synergique existe seulement dans un domaine de concentrations relatives de chacun de ces deux excipients. L'effet optimal est délimité pour des rapports R avec R représentant la masse mannitol/masse d'alanine présent dans le lyophilisat compris entre 0,1 et 1, notamment 0,2 et 0,8.

De plus, il a été démontré que pour R compris entre 0,1 et 1 :
Le lyophilisat est constitué d'une phase amorphe et d'une phase cristalline.
La phase amorphe est majoritairement constituée de mannitol et de protéine.
La phase cristalline est majoritairement constituée d'alanine

Les hypothèses envisagées sont que pour R compris entre 0,1 et 1 :
La phase amorphe constituée cryoprotège la protéine en cours de congélation.
La phase cristalline fixe la structure du lyophilisat et évite son effondrement.

C'est cet effet synergique surprenant entre la coexistence d'une phase amorphe et d'une phase cristalline qui stabilise la protéine lyophilisée. La présente invention décrit donc l'obtention de cet effet pour des rapports R privilégiés.

Ainsi la présente invention concerne des formes pharmaceutiques lyophilisées contenant une quantité efficace d'une protéine biologiquement active, un tampon ajusté au pH optimal de stabilité de la protéine, de l'alanine et du mannitol, ces deux derniers excipients étant dans un rapport massique R = masse de mannitol/masse d'alanine compris entre 0,1 et 1. La protéine incluse dans la dite formulation reste stable sous forme lyophilisée. La dissolution du lyophilisat obtenu est rapide et totale. L'aspect du lyophilisat n'est pas effondré et sa teneur en eau est compatible avec le maintien de l'activité de la protéine.

D'autres excipients pharmaceutiquement acceptables, bien connus de l'homme de l'art, peuvent être introduits dans cette formulation, comme par exemple des co-solvants, des conservateurs, des anti-oxydants ou des agents chélatants. Le tampon utilisé est préférentiellement un tampon phosphate.

L'objet de la présente invention consiste donc à obtenir des lyophilisats stables contenant une protéine cryoprotégée par une phase solide amorphe en cours de congélation constituée essentiellement de la protéine et du mannitol, cette phase amorphe coexistant au sein du lyophilisat obtenu après sublimation et dessiccation de la solution congelée, avec une phase cristalline constituée essentiellement par l'alanine.

La protéine biologiquement active (ou bioactive) qui est formulée selon la présente invention peut être un polypeptide glycosylé ou non, naturel, synthétique, semisynthétique ou recombinant tel qu'utilisé dans la pratique clinique ou de laboratoire. Plus particulièrement, ladite protéine peut être par exemple une hormone comme une hormone de croissance, de préférence l'hormone de croissance humaine (hGH), une hormone lutéinique (LH-RH), une gonadotrophine. La protéine peut également être une enzyme, par exemple une enzyme thrombolytique telle qu'une urokinase, une pro-urokinase, une streptokinase, une staphilokinase, un activateur tissulaire du plasminogène (tPA) ou une enzyme telle qu'une phosphatase, une sulfatase, une acyl-transférase, une mono-amino oxydase, une urate oxydase. De même, une protéine qui est formulée selon la présente invention peut être une cytokine, comme par exemple l'interleukine-2 (IL-2), l'interleukine-4 (IL-4), l'interleukine-6 (IL-6) ou l'interleukine-13 (IL-13). Une autre classe de protéines selon la présente intervention inclut par exemple les anticorps, les immunoglobulines, les immunotoxines. Des peptides tels que la cholecystokinine (CCK), la substance P, la neurokinine A, la neurokinine B, la neurotensine, le neuropeptide Y, l'elédoïsine, la bombésine peuvent être formulés selon la présente invention.

La protéine biologiquement active est de préférence l'hGH (ou hormone de croissance humaine), l'urate oxydase ou l'interleukine-13.

L'hormone de croissance humaine est une protéine formée d'une seule chaîne polypeptidique de 191 acides aminés avec 2 ponts disulfures entre les résidus cystéine 53 et 165 et les résidus cystéine 182 et 189.

L'urate oxydase est une enzyme qui oxyde l'acide urique en allantoine et est extraite de la biomasse d'*Aspergillus flavus* (Laboureur et al., Bull. Soc. Chim. Biol., 1968, *50*, 811-825). Elle a été utilisée pour le traitement des hyperuricémies depuis plus de 20 ans.

L'ADNc codant pour cette protéine a été cloné récemment et exprimé dans *E*. *coli* (LEGOUX R et al., J. of Biol. Chem., 1992, *267*, 12, 8565-8570), *Aspergillus flavus* et *Saccharomyces cerevisiae.* L'enzyme est un tétramère avec des sous unités identiques de masse moléculaire voisines de 32000. Le monomère, formé d'une seule chaîne polypeptidique de 301 acides aminés, n'a pas de ponts disulfures et est acétylé à l'extrémité N-terminale.

L'interleukine-13 est une cytokine constituée d'une seule chaîne polypeptidique de 112 acides aminés avec deux ponts disulfure (Minty et al., Nature, 1993, *362*, 248-250).

L'obtention de la coexistence de la phase (mannitol + protéine) amorphe avec la phase alanine cristalline est indépendante de la présence et de la concentration d'un tampon pour ajuster le pH de la solution, mais elle dépend du rapport R précédemment défini.

Afin de proposer des formulations de protéines disponibles pour le public en tant qu'agents thérapeutiques, il est indispensable de les formuler sous une forme suffisamment stable de façon à maintenir leur activité biologique entre le moment de la formulation et celui de l'utilisation. L'hGH, par exemple, a été formulée de nombreuses manières telles que celles décrites dans les brevets ou demandes de brevet suivants : US 5,096,885 ; WO 89/09614 ; WO 92/17200 ; Au-30771/89 ; WO 93/19773 ; WO 93/19776. Ce dernier document décrit des formulations d'hGH dans un tampon citrate, contenant de la glycine ou du mannitol et/ou du glycérol. Il est indiqué que les solutions d'hormone de croissance contenant du citrate sont plus stables que celles préparées à base de tampon phosphate.

Les formulations selon l'invention peuvent être conservées à température ambiante, alors que les formulations contenant ces protéines commercialisées à l'heure actuelle doivent être conservées à des températures de 2°C à 8°C.

Dans la plupart des cas la forme pharmaceutique est lyophilisée, congelée, ou en solution. Elle contient la protéine, un tampon, de la glycine, de l'arginine, du mannitol, du zinc, des tensioactifs, du dextran, de l'EDTA, ou d'autres excipients mais jamais l'association alanine/mannitol dans un rapport massique R compris entre 0,1 et 1. Les formes lyophilisées ou congelées sont utilisées pour maintenir l'intégrité biochimique et l'activité biologique de la molécule. Les formulations lyophilisées doivent être reconstituées avant utilisation par addition de solvants stériles pharmaceutiquement acceptables comme de l'eau distillée, des solutions aqueuses de chlorure de sodium à 0,9 % ou de glucose à 5 % ou tout autre solvant physiologiquement acceptable, contenant ou non des conservateurs antibactériens tels que l'alcool benzylique, le phénol ou le métacrésol.

Une formulation stable à température ambiante jusqu'à sa reconstitution est particulièrement avantageuse pour un traitement ambulatoire comme dans le cas de l'hGH sous forme de flacon ou de présentation adaptée sous forme de stylo multidose.

La formulation ainsi préparée peut également être introduite dans une trousse de dosage.

La présente invention est donc une composition privilégiée d'un lyophilisat. Ce lyophilisat est obtenu par lyophilisation à partir d'une solution.

Le procédé de cette formulation inclus des étapes de mélange, de dissolution, de filtration et de lyophilisation.
La composition de la solution à lyophiliser est la suivante :

Une protéine, un tampon pharmaceutiquement acceptable pour régler le pH, de l'alanine, du mannitol où le rapport massique R = masse de mannitol/masse d'alanine est compris entre 0,1 et 1, de l'eau pour préparation injectable.
La solution à lyophiliser se prépare de la façon suivante :

La solution de protéine est obtenue sur une colonne de Gel-filtration et contient un tampon maintenant son pH dans une zone compatible avec la stabilité de la protéine.

A cette solution on ajoute les quantités désirées de tampon, d'alanine, de mannitol et d'eau de façon à solubiliser l'ensemble des excipients. La solution est filtrée stérilement et répartie en récipients, préférentiellement des flacons ou des carpules.
La lyophilisation des solutions est réalisée comme suit :

La solution suit un cycle de congélation, puis de sublimation et de dessiccation adapté au volume à lyophiliser et au récipient contenant la solution. Préférentiellement on choisit une vitesse de congélation proche de -2°C/mn dans un lyophilisateur Usi-froid (France) de type SMH15 ou SMJ100 ou SMH2000.

Le temps, la température et la pression de dessiccation du lyophilisat sont ajustés en fonction des volumes de solution à lyophiliser et de la teneur en eau résiduelle désirée dans le lyophilisat.

Une information complète sur les techniques de préparation des formulations injectables est à la disposition de l'homme du métier dans Remington's Pharmaceutical Sciences, 1985, 17th Edition ou dans William NA & Polli GP, The lyophilization of pharmaceuticals : a litterature review, J. Parenteral Sci. Tech., 1984, *38*, (2), 48-59 ou dans Franks F., Freeze-drying : from enpiricism to predictability, Cryo-letters, 1990, *11*, 93-110.

On obtient alors un lyophilisat dans lequel l'alanine se trouve sous forme cristallisée, et le mannitol sous forme amorphe. Le lyophilisat peut être conservé à 25°C sans altérer l'activité biologique de la protéine qu'il contient.

Afin d'illustrer la présente invention, des évaluations ont été effectuées en choisissant comme exemple de protéine l'hGH ou l'urate oxydase. Ainsi plusieurs solutions contenant l'hGH ou de l'urate oxydase comme protéine biologiquement active, un tampon phosphate à différentes concentrations, à pH = 7 pour les solutions contenant de l'hGH (à 4 UI/0,5 ml), et pH = 8 pour celles contenant l'urate oxydase (à 30 UAE/ml, du mannitol seul, de l'alanine seule, ou des mélanges alanine/mannitol) ont été préparées, lyophilisées et analysées.

Les compositions sont décrites en détail dans les exemples des TABLEAUX 1 et 2 ci-dessous. Les méthodes d'analyse ainsi que les temps et les températures de mise en stabilité sont également décrits ci-après.

**TABLEAU 1**

| Le TABLEAU 1 ci-dessous indique les compositions des formules étudiées contenant de l'urate oxydase. | | | | | |
|---|---|---|---|---|---|
| Lot n° | mg Mannitol | mg Alanine | R | Tampon phosphate pH=8 ; mM | Urate Oxydase UAE⁽¹⁾ |
| 1 | 33,0 | 0,0 | + ∞ | 50 | 30 |
| 2 | 27,7 | 6,9 | 4 | 30 | 30 |
| 3 | 20,8 | 10,4 | 2 | 30 | 30 |
| 4 | 13,6 | 13,6 | 1,000 | 40 | 30 |
| 5 | 10,2 | 15,3 | 0,67 | 40 | 30 |
| 6 | 7,3 | 16,0 | 0,46 | 40 | 30 |
| 7 | 7,3 | 16,2 | 0,45 | 30 | 30 |
| 8 | 9,5 | 21,0 | 0,45 | 50 | 30 |
| 9 | 4,6 | 18,3 | 0,25 | 40 | 30 |
| 10 | 2,5 | 20,0 | 0,125 | 40 | 30 |
| 11 | 0,0 | 16,0 | 0 | 50 | 30 |

| | | | | | |
|---|---|---|---|---|---|
| ⁽¹⁾ : UAE signifie unité d'activité enzymatique | | | | | |

**TABLEAU 2**

| Le TABLEAU 2 ci-dessous indique la composition des formules étudiées contenant de l'hGH | | | | | |
|---|---|---|---|---|---|
| Lot n° | mg Mannitol | mg Alanine | R | Tampon phosphate pH=7 ; mM | hGH UI⁽¹⁾ |
| 12 | 12,5 | 0,0 | + ∞ | 2,50 | 4 |
| 13 | 20,0 | 2,5 | 8 | 1,95 | 4 |
| 14 | 12,5 | 6,5 | 2 | 0,00 | 4 |
| 15 | 12,5 | 6,5 | 2 | 2,50 | 4 |
| 16 | 12,5 | 13,0 | 1 | 2,50 | 4 |
| 17 | 4,7 | 10,5 | 0,45 | 1,95 | 4 |
| 18 | 0,0 | 6,5 | 0 | 2,50 | 4 |

| | | | | | |
|---|---|---|---|---|---|
| ⁽¹⁾ : UI : Unité Internationale | | | | | |

Les méthodes analytiques qui ont été utilisées pour la détermination des différents paramètres sont les suivantes.

### Teneurs en dimères et substances apparentées de masse moléculaire plus élevées :

La teneur en dimères et substances apparentées de masse moléculaire plus élevée est déterminée par chromatographie d'exclusion (SEC-HPLC) en utilisant une colonne SUPEROSE 12 (Pharmacia, Réf. 17-0538-01). Le produit est élué avec une solution tampon phosphate d'ammonium à pH = 7,0 (1,38 g de dihydrogénophosphate d'ammonium dans 1 litre d'eau, ajusté à pH = 7,0 avec de l'ammoniaque concentré à un débit de 0,4 ml/minute). La détection est réalisée à 220 nm. (Cette teneur est notée dans les résultats analytiques en pourcentages d'oligomères + polymères).

La teneur en dimère et substances de masse moléculaire plus élevée peut également être déterminée par la méthode décrite dans la monographie de la Pharmacopée Européenne "Somatropine pour préparation injectable" de janvier 1994.

### Dosage du titre en protéine par chromatographie sur phase inverse.

Exprimée en mg par flacon il est déterminé par chromatographie sur phase inverse en utilisant une colonne C18-300Å - 25 cm, diamètre 4,6 mm (SYNCHROM, Réf. CR103-25). Le produit est élué en 35 minutes en mode gradiant avec une phase mobile passant de 75 volumes d'eau à 0,1 % d'acide trifluoroacétique (V/V) (TFA) et 25 volumes d'acétonitrile à 0,08 % de TFA (V/V) à 30 volumes d'eau à 0,1 % de TFA et 70 volumes d'acétonitrile à 0,08 % TFA. Le débit est de 1 ml par minute et la détection est réalisée à 220 nm.

### Dosage de l'activité enzymatique de l'urate oxydase.

L'activité enzymatique de l'urate oxydase exprimée en UAE est déterminée par spectrophotométrie dans une cuve thermostatée à 30°C en suivant la disparition de l'acide urique à 292 nm selon Legoux R, Delpech Bruno, Dumont X, Guillemot JC, Ramond P, Shire D, Caput D, Ferrara P, Loison G, J. Biol. Chem., 1992, *267 (12)*, 8565-8570.

### Turbidité des solutés reconstitués.

La turbidité des lyophilisats contenant de l'hGH repris en solution est déterminée par spectrophotométrie (Ph. Eur. 2 (I) V.619) à 500 nm sur un spectrophotomètre Perkin Elmer 554. Les résultats sont fournis en Unités d'Absorbance x 1000.

La turbidité des lyophilisats contenant de l'urate oxydase repris en solution est déterminée à l'aide d'un turbidimètre Ratio Hach 18900-00. Les résultats de turbidité sont exprimés en Unités Néphélométriques de Turbidité (NTU) définie par : Standard methods for the examination of water and wastewater de l'American Public Health Association.

Le degré d'opalescence est également déterminé selon la méthode de la Pharmacopée Européenne (II) V. 6, par comparaison de l'échantillon à analyser avec une suspension témoin.

### Critères organoleptiques des lyophilisats.

Ces critères sont examinés visuellement et prennent en compte la coloration du lyophilisat, sa structure (effondré ou non), ainsi que l'observation d'un déphasage éventuel entre la croûte et la mie du lyophilisat.

### Diffractométrie des Rayons X sur poudre.

L'analyse diffractométrique des Rayons X sur les lyophilisats est effectuée sur un diffractomètre SIEMENS D500 TT ; Source : CuKαl ; Générateur : 40 KV, 25 mA ; Monochromateur arrière ; Fentes : 1/1/1/0,16/0,6 ; Echantillonnage sur portoir pyrex ; Domaine de balayage : 4° à 40° par minute en 2 thêta de Bragg.

### Analyse thermique différentielle.

L'étude des lyophilisats par analyse thermique différentielle est effectuée dans les conditions suivantes :

Appareillage : DSC 7 Perkin Elmer ; Etalonnage : Indium et Plomb ; Prise d'essai : entre 5 mg et 10 mg dans une capsule de 50 µl ; Température initiale : 10°C ; Vitesse de chauffage 10°C/minute ; température finale : 300°C.

### Formes déamidées de l'hGH.

Le pourcentage de formes déamidées est déterminé par chromatographie d'échange anionique (AEX-HPLC) en utilisant une colonne échangeuse d'anions (PHARMACIA mono-Q HR 5/5, Réf. 17-0546-01). L'élution est réalisée avec une solution A (13,8 g de dihydrogénophosphate d'ammonium dans 1 000 ml d'eau ; pH = 7 ajusté avec l'ammoniaque concentrée) et de l'eau comme solution B en utilisant la programmation suivante : 5 % de solution A pendant 2 minutes, puis passage à 15 % de solution A en 5 minutes, ensuite passage à 50 % de solution A en 20 minutes, enfin passage à 100 % de solution A en 5 minutes et maintient de cette dernière solution pendant 5 minutes. L'élution de l'hGH (tr environ 15 minutes) et des formes déamidées est suivie à 220 nm. Le débit est de 1 ml par minute.
Les résultats analytiques obtenus en utilisant ces différentes méthodes sont décrits ci-après.

### Teneur en dimères et substances apparentées de masse moléculaire plus élevée

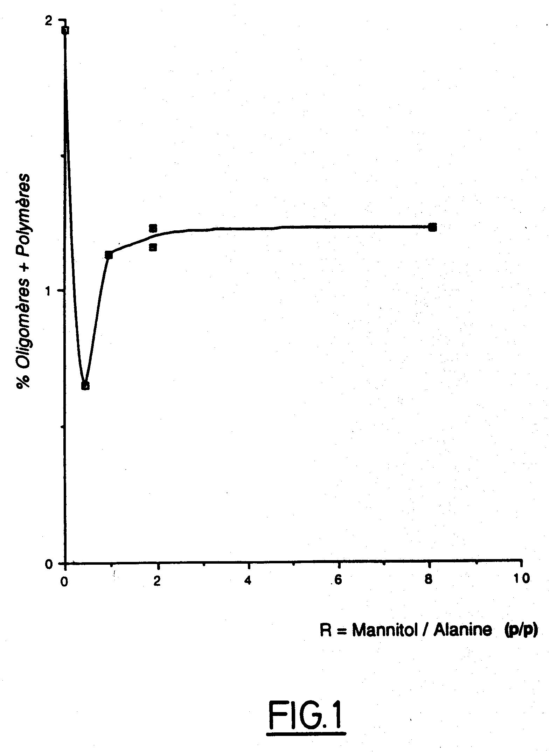

La teneur en oligomères plus polymères des lyophilisats contenant 4 UI d'hGH repris par 0,5 ml d'eau a été déterminée en fonction du rapport R (Figure 1). La Figure 1 indique que la teneur en oligomères plus polymères minimale des solutions est obtenue pour R compris entre 0,1 et 1, la valeur 0,1 étant interpolée à partir de la courbe.

A titre d'exemple supplémentaire deux lots d'hGH à 4UI et 8UI ont été suivis en stabilité à 25°C et 35°C. Leur stabilité est excellente après 6 mois de conservation.

Les TABLEAUX 3 et 4 ci-après indiquent les pourcentages d'oligomères et de polymères de formes déamidées à différents temps et températures en comparaison avec les normes de la Pharmacopée Européenne.
Dans ces tableaux, R = 0,45.

**TABLEAU 3**

| Lot 4UI par flacon | | | | | |
|---|---|---|---|---|---|
| | Norme Pharmacopée Européenne | Temps 0 | à 25°C | à 35°C | |
| | | | 3 mois | 3 mois | 6 mois |
| Oligomères et polymères en % | 6,0 | 1,0 | 1,9 | 3 | 3,3 |
| Formes déamidées en % | 6,6 | 1,3 | 1,9 | 2,8 | 3,9 |
| Dosage en mg par flacon | - | 1,58^{(*)} | 1,61^{(*)} | 1,54^{(*)} | 1,55^{(*)} |

| | | | | | |
|---|---|---|---|---|---|
| ^{(*)} : Le titre ne varie pas significativement au cours du temps compte tenu de la précision de la méthode de dosage (± 5 %) | | | | | |

**TABLEAU 4**

| Lot 8UI par flacon | | | | | | |
|---|---|---|---|---|---|---|
| | Norme Pharmacopée Européenne | Temps 0 | à 25°C | | à 35°C | |
| | | | 3 mois | 6 mois | 3 mois | 6 mois |
| Oligomères et polymères en % | 6 | 1,0 | 1,5 | 1,2 | 1,7 | 2,2 |
| Formes déamidées en % | 6,6 | 1,0 | 1,5 | 1,95 | 2,2 | 3,3 |
| Dosage en mg par flacon | - | 3,0^{(*)} | 3,0^{(*)} | 2,93^{(*)} | 2,95^{(*)} | 2,94^{(*)} |

| | | | | | | |
|---|---|---|---|---|---|---|
| ^{(*)} : Le titre ne varie pas significativement au cours du temps compte tenu de la précision de la méthode de dosage (± 5 %) | | | | | | |

Dosage de l'activité enzymatique de l'urate oxydase.

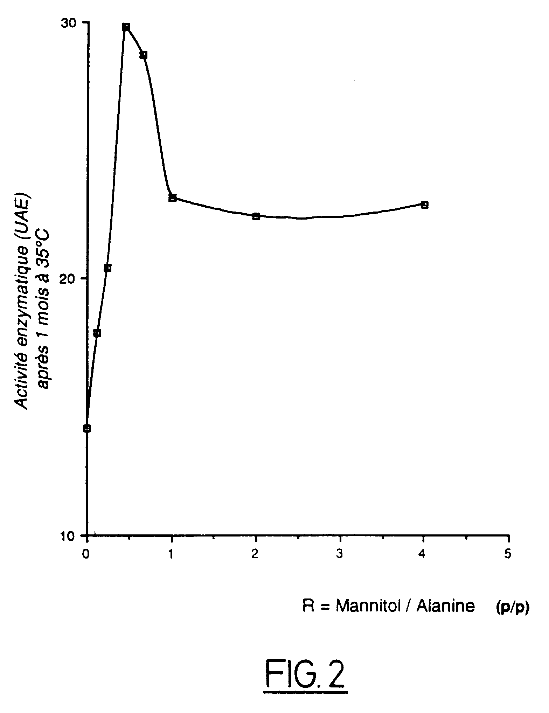

L'activité enzymatique de lyophilisats contenant 30 UAE d'urate oxydase repris par 1 ml d'eau a été déterminée en fonction du rapport R après 1 mois à 35°C (cf Figure 2). La Figure 2 indique que l'activité enzymatique initiale de l'urate oxydase est conservée après 1 mois à 35°C pour R compris entre 0,4 et 1.

A titre d'exemple supplémentaire une formule avec R = 0,45 ou R = 0,67 est parfaitement stable après 3 mois à 25°C (Activité résiduelle voisine de 100 % de celle du temps zéro).

### Turbidité du soluté reconstitué.

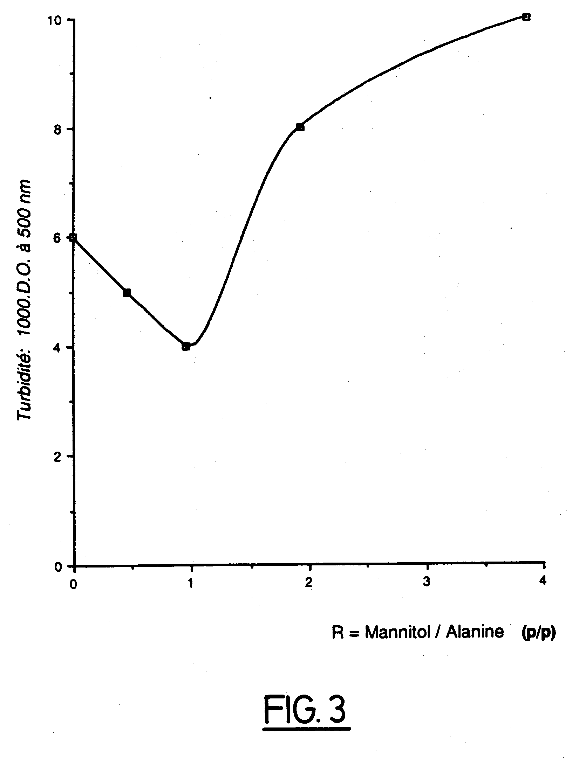

La turbidité des lyophilisats contenant 4 UI d'hGH repris par 0,5 ml d'eau a été déterminée en fonction du rapport R (cf Figure 3). La Figure 3 indique que la turbidité minimale des solutions est obtenue pour R compris entre 0 et 1,5.

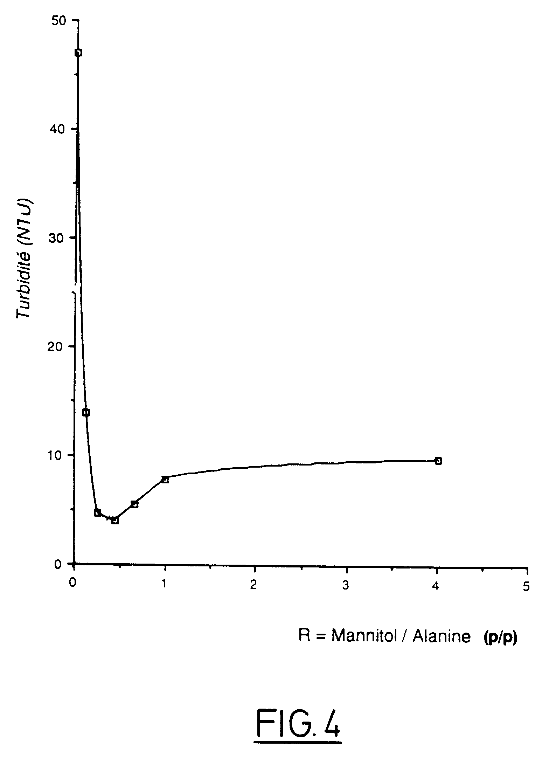

La turbidité des lyophilisats contenant 30 UAE d'urate oxydase repris par 1 ml d'eau a été déterminée en fonction du rapport R (cf Figure 4). La Figure 4 indique que la turbidité minimale obtenue sur les solutions est obtenue pour R compris entre 0,2 et 1.

### Critères organoleptiques des lyophilisats.

Les critères organoleptiques de chaque lyophilisat contenant 4 UI d'hGH ou 30 UAE d'urate oxydase ou aucune protéine, et des teneurs en tampon phosphate variées ont été déterminés en fonction du rapport R, et sont fournis dans le TABLEAU 5. Le TABLEAU 5 indique que les lyophilisats présentent des critères organoleptiques satisfaisants pour R compris entre 0,125 et 1,7. Ces caractères n'évoluent pas en fonction du temps.

**TABLEAU 5**

| Critères organoleptiques des lyophilisats et excipients cristallisés en fonction du rapport R = masse de mannitol/masse d'alanine | | |
|---|---|---|
| R | Critères organoleptiques | Excipients cristallins (Diffraction Rayons X) |
| + ∞ | Effondré | Mannitol |
| 10 | Effondré | Mannitol + Alanine |
| 6,469 | Effondré | Mannitol + Alanine |
| 5 | Effondré | Mannitol + Alanine |
| 4,014 | Effondré | Mannitol + Alanine |
| 2 | Effondré | Mannitol + Alanine |
| 1,705 | Bon | Mannitol + Alanine |
| 1,402 | Bon | Mannitol + Alanine |
| 1,25 | Bon | Mannitol + Alanine |
| 1,097 | Bon | Mannitol + Alanine |
| 1 | Bon | Alanine |
| 0,667 | Bon | Alanine |
| 0,456 | Bon | Alanine |
| 0,456 | Bon | Alanine |
| 0,451 | Bon | Alanine |
| 0,452 | Bon | Alanine |
| 0,251 | Bon | Alanine |
| 0,125 | Bon | Alanine |
| 0 | Déphasage | Alanine |

### Diffraction des rayons X.

Les résultats des analyses par diffraction de rayons X sur la poudre des lyophilisats obtenus contenant des mélanges Alanine/Mannitol dans des rapports R variant de 0 à + ∞ sont fournis dans le TABLEAU 5.

Les diffractogrammes obtenus montrent que pour R compris entre 0 et 1, seules les raies du réseau cristallin de l'alanine apparaissent, de plus l'écart à la ligne de base du diffractogramme indique la présence d'une phase amorphe constituée de mannitol. Plus R augmente et plus la phase amorphe due au mannitol est importante. Pour R > 1 le mannitol cristallise aussi.

On obtient une phase amorphe constituée de mannitol et une phase cristalline constituée d'alanine pour R compris entre 0 et 1.

### Analyse thermique différentielle.

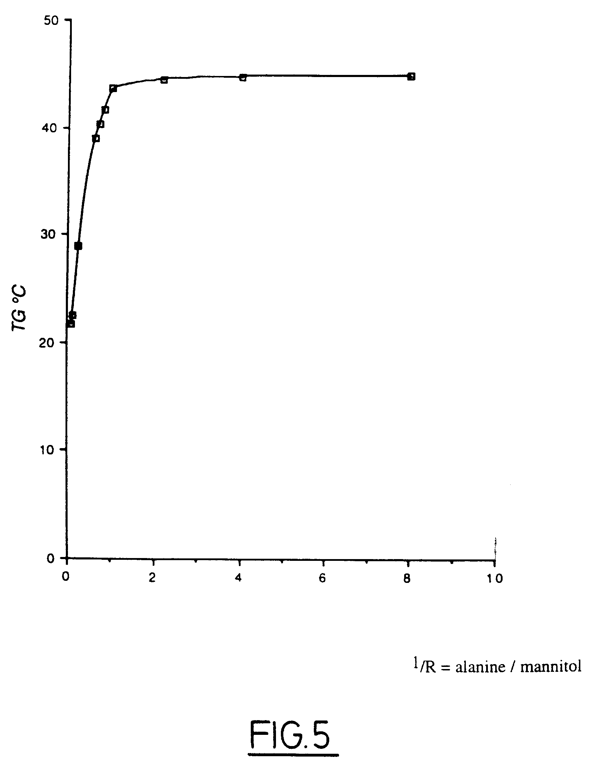

Les températures de transition vitreuse des lyophilisats contenant 4 UI d'hGH ou 30 UAE d'urate oxydase ou aucune protéine, et des teneurs en tampons phosphate variées ont été déterminées en fonction de l'inverse du rapport R (cf Figure 5). La Figure 5 indique que la température de transition vitreuse maximale obtenue sur des lyophilisats contenant des mélanges d'alanine et de mannitol est obtenue pour (1/R) > 1 c'est-à-dire pour R compris entre 0 et 1.

C'est la température de transition vitreuse du lyophilisat qui indique la température maximale de stabilité du lyophilisat. La température maximale de stabilité du lyophilisat est donc atteinte pour R compris entre 0 et 1.

### Formes déamidées

Les résultats obtenus sur les lots 4UI et 8UI ne montrent qu'une très faible évolution qui est très acceptable en comparaison avec les normes de la monographie somatropine de la Pharmacopée Européenne. Ils permettent de penser que cette formule sera suffisamment stable à 25°C.

En conclusion, il a été prouvé que chaque propriété évaluée sur les lyophilisats présente un optimum pour un intervalle de valeur de R qui se résume de la façon suivante :

| | | |
|---|---|---|
| % Oligomères + Polymères (stabilité hGH) Activité enzymatique de l'urate oxydase | R entre | 0,1 et 1 |
| après 1 mois à 35°C | R entre | 0,4 et 1 |
| Turbidité solutions hGH | R entre | 0 et 1,5 |
| Turbidité solutions urate oxydase | R entre | 0,2 et 1 |
| Aspect des lyophilisats | R entre | 0,125 et 1,7 |
| Alanine seule cristalline | R entre | 0 et 1 |
| Température de transition vitreuse maximale | R entre | 0 et 1 |

Chaque critère analytique (% oligomères + polymères, stabilité hGH, activité enzymatique de l'urate oxydase) après 1 mois à 35°C, turbidité solutions hGH, turbidité solutions urate oxydase, aspect des lyophilisats, alanine seule cristalline, température de transition vitreuse maximale) définit un intervalle optimal particulier par propriété. Afin d'obtenir une formulation acceptable on définit un intervalle de R préférentiel, c'est-à-dire 0,1 < R < 1.
Les EXEMPLES suivants illustrent l'invention sans toutefois la limiter.

| **EXEMPLE 1** : Composition d'un lyophilisat d'hGH à 4UI à reprendre par 0,5 ml d'eau PPI | |
|---|---|
| CONSTITUANTS | Formule unitaire en (mg) |
| hGH | 4 UI |
| Alanine | 10,5 mg |
| Mannitol | 4,77 mg |
| Phosphate disodique dodécahydraté | 0,7 mg |
| Eau pour préprarations injectables | QSP 0,5 ml |
| Flacon verre blanc type 1 de 3 ml | 1 |
| Bouchon pilier en chlorobutyle gris | 1 |
| Capsule alu Flip-off bleue diamètre 13 mm | 1 |

| **EXEMPLE 2** : Composition d'un lyophilisat d'hGH à 8UI à reprendre par 1 ml d'eau PPI | |
|---|---|
| CONSTITUANTS | Formule unitaire en (mg) |
| hGH | 8 UI |
| Alanine | 21 mg |
| Mannitol | 9,54 mg |
| Phosphate disodique dodécahydraté | 1,4 mg |
| Eau pour préprarations injectables | QSP 1 ml |
| Flacon verre blanc type 1 de 3 ml | 1 |
| Bouchon pilier en chlorobutyle gris | 1 |
| Capsule alu Flip-off bleue diamètre 13 mm | 1 |

| **EXEMPLE 3** : Composition d'un lyophilisat d'hGH à 18UI à reprendre par 1 ml d'eau PPI | |
|---|---|
| CONSTITUANTS | Formule unitaire en (mg) |
| hGH | 18 UI |
| Alanine | 21 mg |
| Mannitol | 9,54 mg |
| Phosphate disodique dodécahydraté | 1,4 mg |
| Eau pour préprarations injectables | QSP 1,0 ml |
| Flacon verre blanc type 1 de 3 ml | 1 |
| Bouchon pilier en chlorobutyle gris, diamètre 13 mm | 1 |
| Capsule aluminium operculée, diamètre 13 mm | 1 |

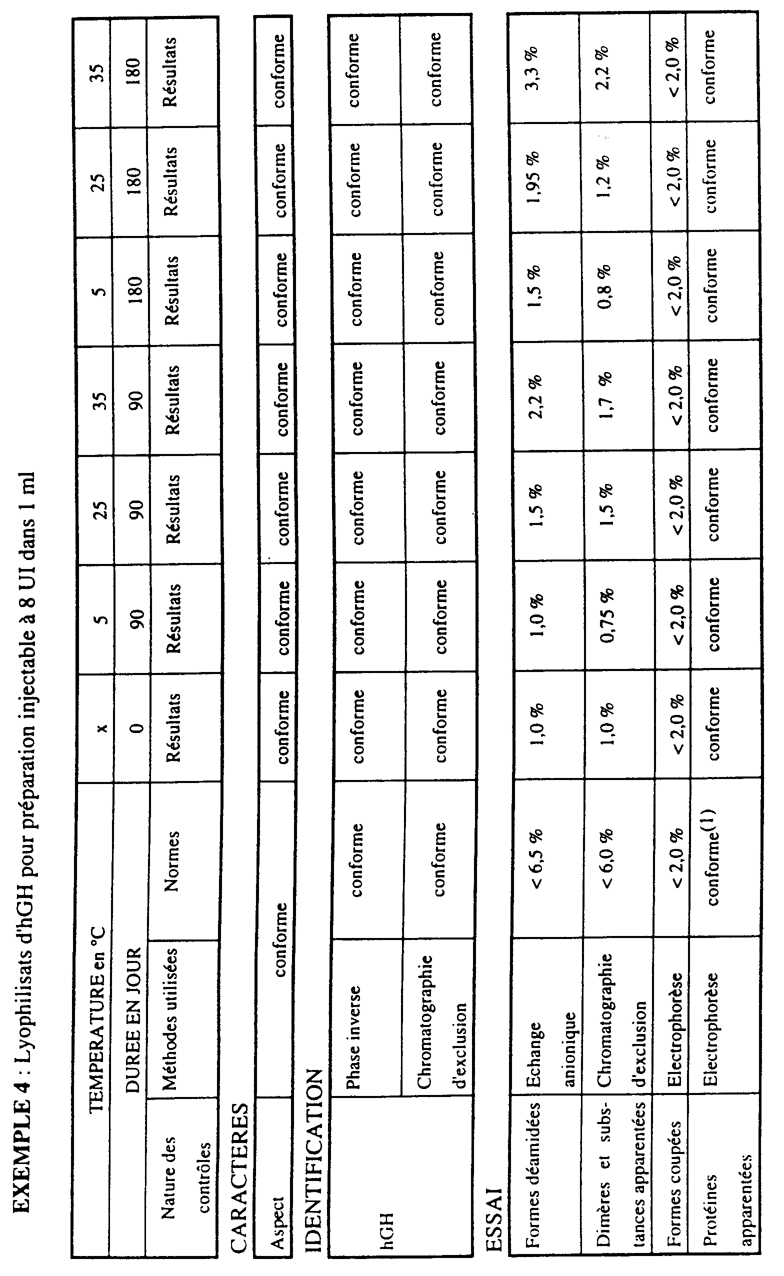

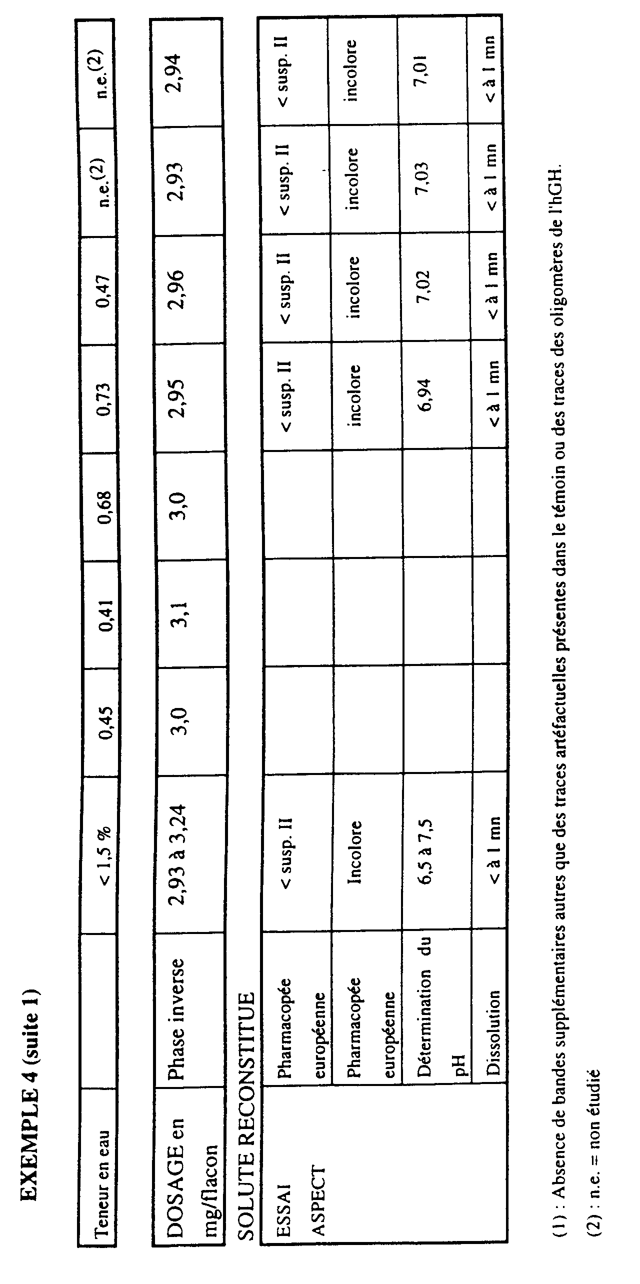

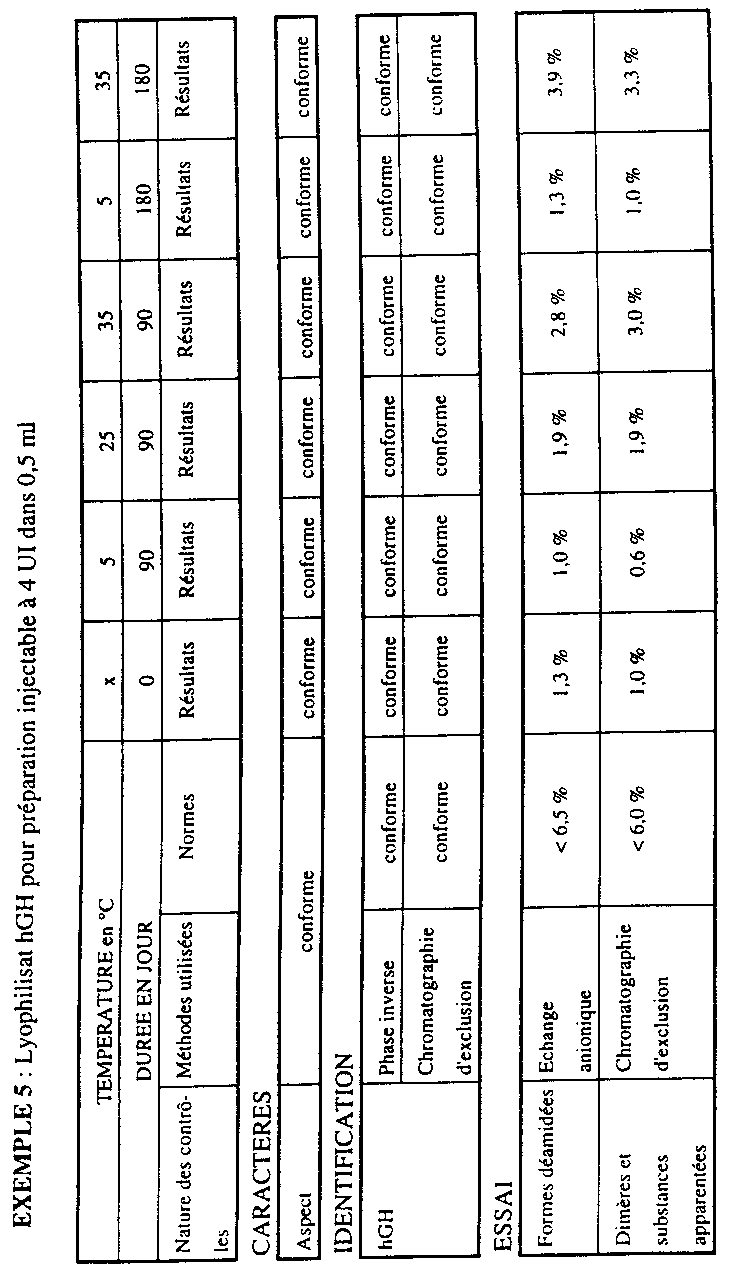

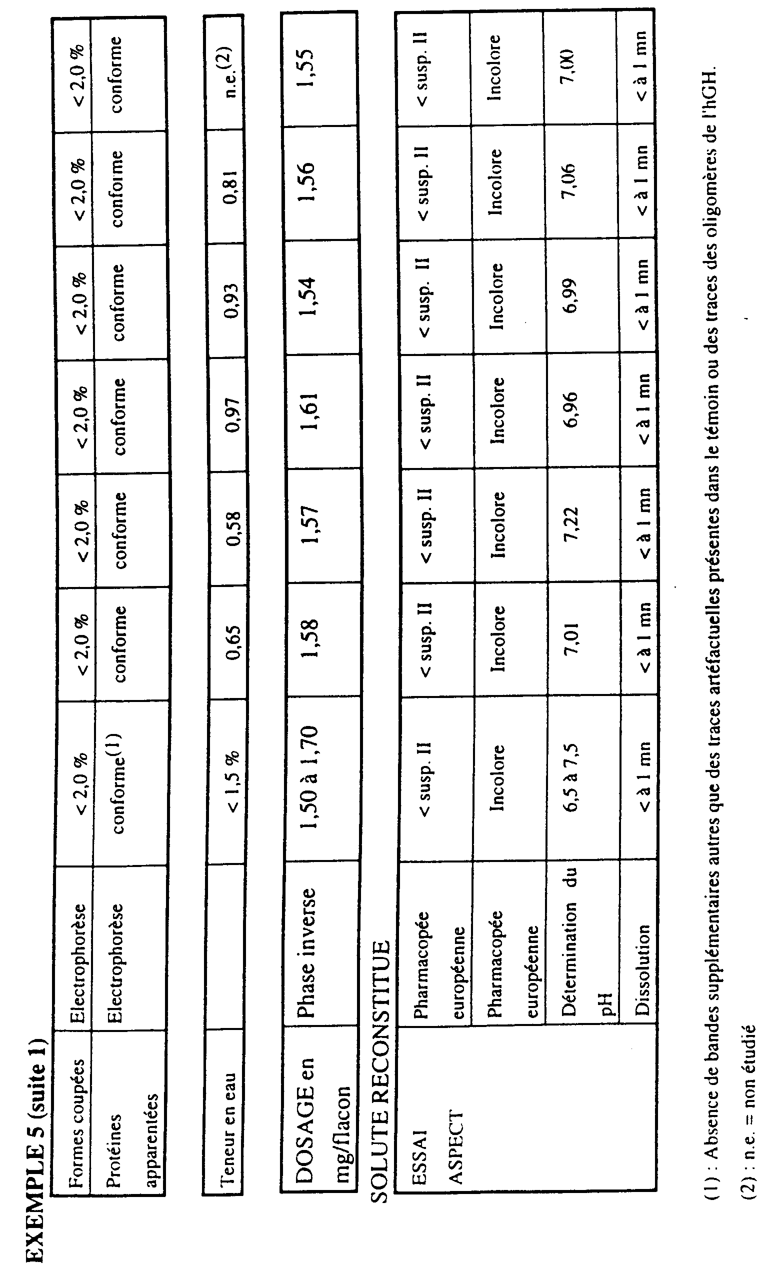

| **EXEMPLE 6** : Résultats analytiques après 1 an de stabilité à 5°C d'hGH à 18 UI | | | | | |
|---|---|---|---|---|---|
| | Opalescences (U.A. x 10⁻³) | pH | Teneur en eau | Formes déamidées | Oligomères + Polymères |
| normes | < 20 | | < 1,5 % | < 5 % | < 6 % |
| t = 0 | 1 | 7,9 | 0,33 % | 1,36 % | 0,63 % |
| 1 mois | 1 | 8 | 0,29 % | - | - |
| 3 mois | 3 | 7,83 | 0,32 % | 1,25 % | 0,45 % |
| 6 mois | 4 | - | 1,03 % | 1,4 % | 0,5 % |
| 12 mois | 7 | - | 1,19 % | 1,15 % | 1,11 % |
| Pour l'ensemble des résultats, R = 0,45 | | | | | |

| **EXEMPLE 7** : Résultats analytiques après 3 mois de stabilité à 25°C d'hGH 18 UI | | | | | |
|---|---|---|---|---|---|
| | Opalescences (U.A. x 10⁻³) | pH | Teneur en eau | Formes déamidées | Oligomères + Polymères |
| normes | < 20 | | < 1,5 % | < 5 % | < 6 % |
| t = 0 | 1 | 7,9 | 0,33 % | 1,36 % | 0,63 % |
| 1 mois | 3 | 7,72 | 0,35 % | - | - |
| 3 mois | 9 | 7,65 | 0,54 % | 1,37 % | 0,85 % |
| Pour l'ensemble des résultats, R = 0,45 | | | | | |

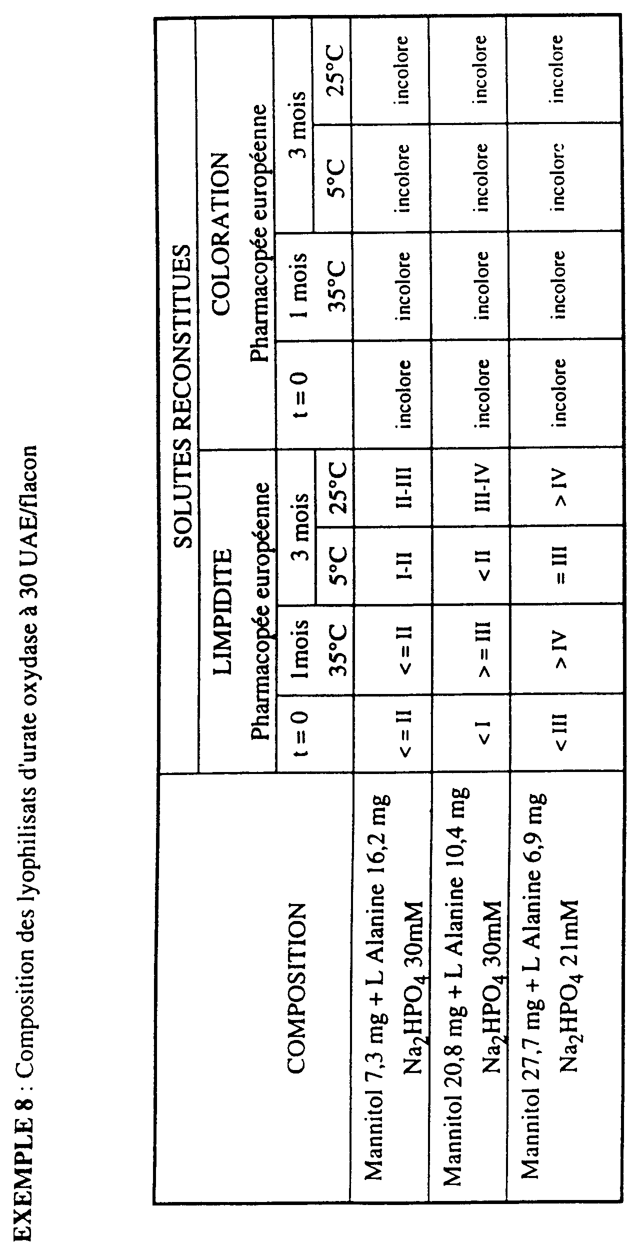

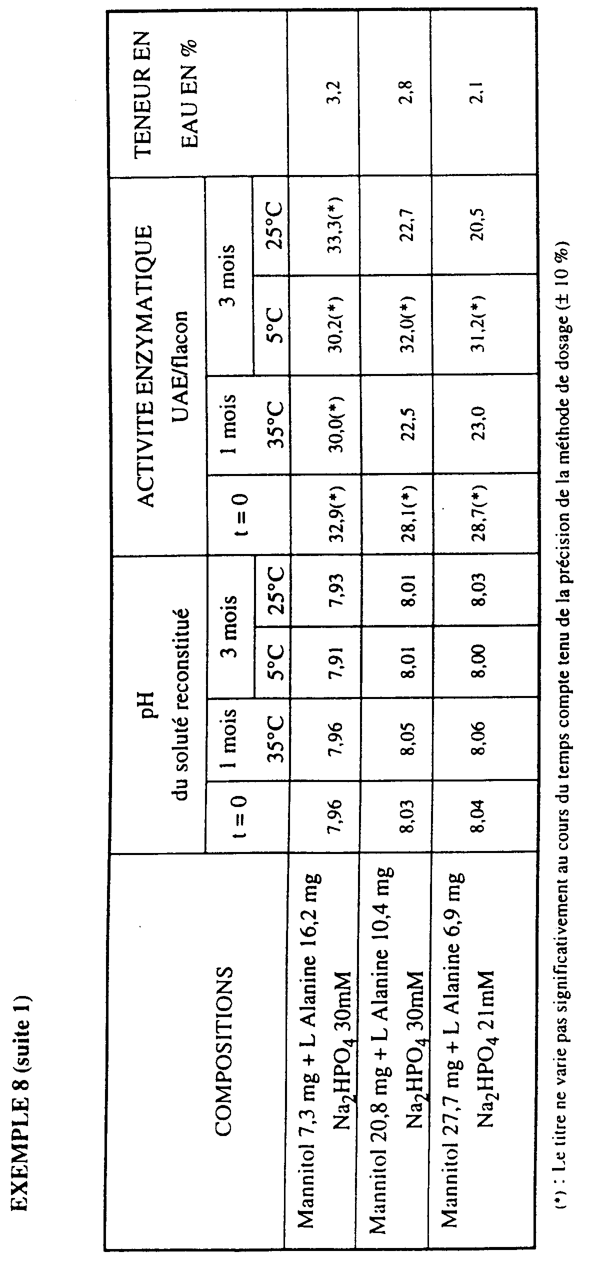

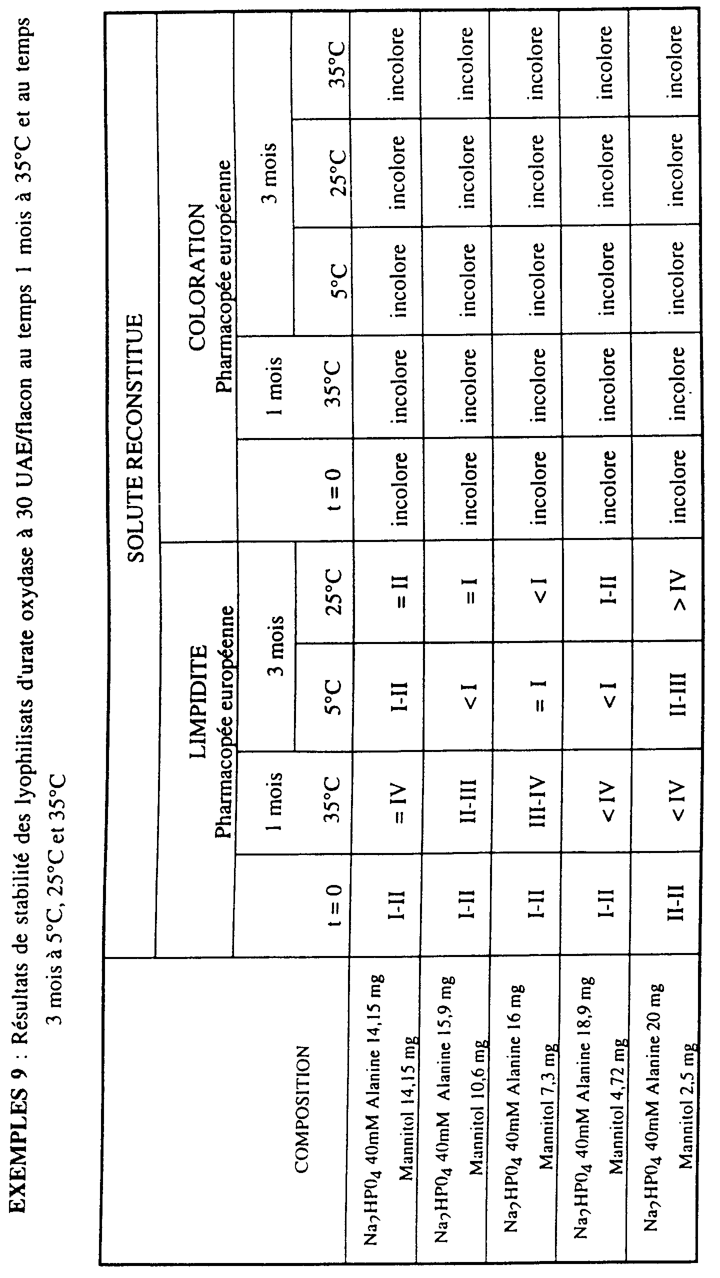

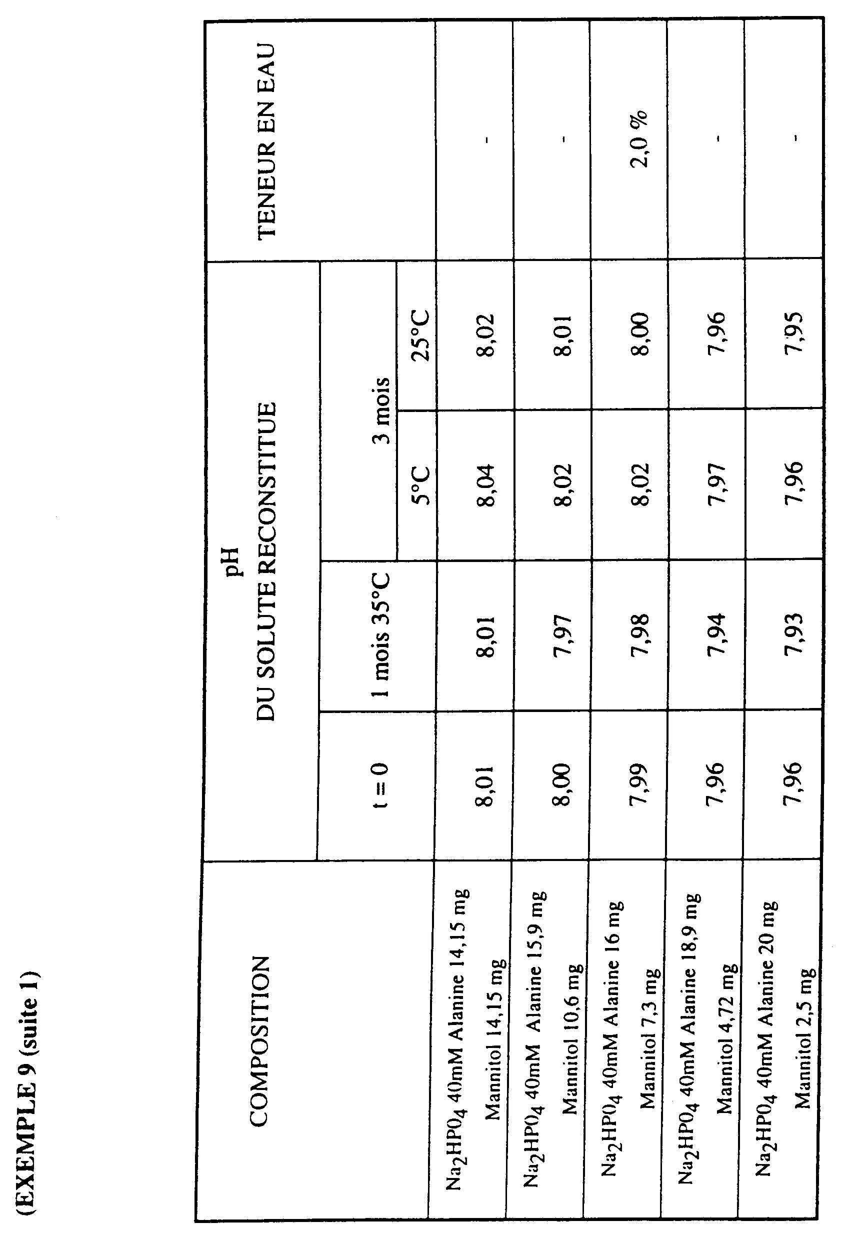

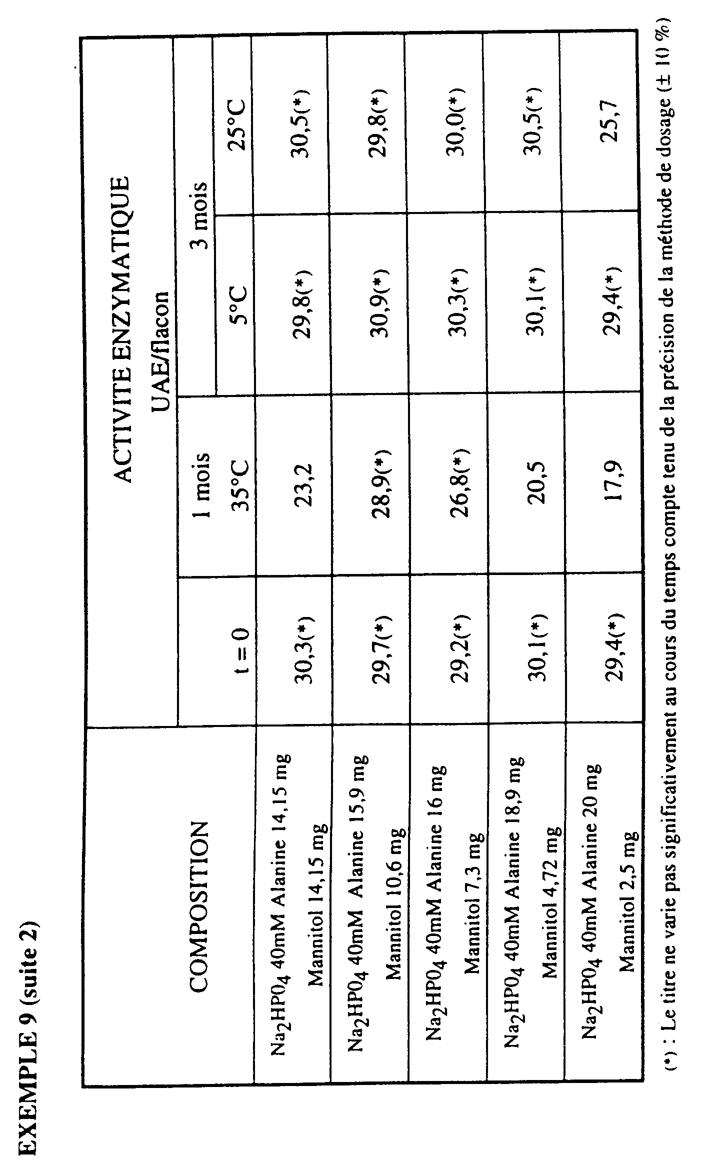

## Revendications

1. Formulation stable lyophilisée pharmaceutiquement acceptable d'une protéine, cette formulation étant constituée de la protéine, d'un tampon, de l'alanine et du mannitol, dans laquelle le rapport R = masse de mannitol/masse d'alanine est compris entre 0,1 et 1.

2. Formulation selon la revendication 1, dans laquelle la protéine est une hormone, une enzyme ou une cytokine.

3. Formulation selon la revendication 1 pour la reconstitution d'une solution injectable.

4. Formulation selon la revendication 1 pour la reconstitution d'une solution injectable à l'homme ou à l'animal par voie sous-cutanée, intraveineuse, ou intramusculaire.

5. Formulation selon la revendication 2 dans laquelle l'hormone est l'hGH.

6. Formulation selon la revendication 2 dans laquelle l'enzyme est l'urate oxydase.

7. Formulation selon la revendication 2 dans laquelle la cytokine est l'interleukine-13.

8. Formulation selon la revendication 1 où l'alanine est sous forme cristallisée et le mannitol sous forme amorphe.

9. Formulation selon la revendication 1, caractérisée en ce que le lyophilisat est constitué d'une phase amorphe et d'une phase cristalline, la phase amorphe étant majoritairement constituée de mannitol et de protéine, la phase cristalline étant majoritairement constituée d'alanine.

10. Formulation selon la revendication 2, dans laquelle le tampon est un tampon phosphate.

11. Kit de dosage caractérise en ce qu'il comprend une formulation selon la revendication 1.

## Claims

1. Pharmaceutically acceptable stable lyophilised formulation of a protein, this formulation consisting of the protein, a buffer, alanine and mannitol, wherein the ratio R = mass of mannitol/mass of alanine is between 0.1 and 1.

2. Formulation according to claim 1, wherein the protein is a hormone, an enzyme or a cytokine.

3. Formulation according to claim 1 for reconstituting an injectable solution.

4. Formulation according to claim 1 for reconstituting a solution which can be injected into humans or animals by subcutaneous, intravenous or intramuscular route.

5. Formulation according to claim 2 wherein the hormone is hGH.

6. Formulation according to claim 2 wherein the enzyme is urate oxidase.

7. Formulation according to claim 2 wherein the cytokine is interleukin-13.

8. Formulation according to claim 1 wherein the alanine is in crystalline form and the mannitol is in amorphous form.

9. Formulation according to claim 1, characterised in that the lyophilisate consists of an amorphous phase and a crystalline phase, the amorphous phase consisting chiefly of mannitol and protein and the crystalline phase consisting chiefly of alanine.

10. Formulation according to claim 2, wherein the buffer is a phosphate buffer.

11. Titration kit, characterised in that it comprises a formulation according to claim 1.

## Patentansprüche

1. Stabile, gefriergetrocknete pharmazeutisch annehmbare Formulierung eines Proteins, welche Formulierung aus Protein, einem Puffer, Alanin und Mannit gebildet ist, worin das Verhältnis R = Masse Mannitol/Masse Alanin zwischen 0,1 und 1 liegt.

2. Formulierung nach Anspruch 1, worin das Protein ein Hormon, ein Enzym oder ein Cytokin ist.

3. Formulierung nach Anspruch 1 zur Bildung einer injizierbaren Lösung.

4. Formulierung nach Anspruch 1 zur Bildung einer am Menschen oder Tier auf subcutanem, intravenösem oder intramuskulärem Wege injizierbaren Lösung.

5. Formulierung nach Anspruch 2, worin das Hormon hGH ist.

6. Formulierung nach Anspruch 2, worin das Enzym Urat-oxidase ist.

7. Formulierung nach Anspruch 2, worin das Cytokin Interleukin-13 ist.

8. Formulierung nach Anspruch 1, worin Alanin in kristalliner Form und Mannit in amorpher Form vorliegen.

9. Formulierung nach Anspruch 1, dadurch gekennzeichnet, daß das gefriergetrocknete Material aus einer amorphen Phase und einer kristallinen Phase gebildet ist, wobei die amorphe Phase überwiegend aus Mannit und Protein gebildet ist und die kristalline Phase überwiegend aus Alanin gebildet ist.

10. Formulierung nach Anspruch 2, worin der Puffer ein Phosphatpuffer ist.

11. Dosierungskit, dadurch gekennzeichnet, daß er eine Formulierung nach Anspruch 1 umfaßt.
